# EUROPEAN PATENT SPECIFICATION

(11) **EP 0 667 888 B1**
(45) Date of publication and mention of the grant of the patent: **12.02.1997**
(21) Application number: 93923023.1
(22) Date of filing: 20.10.1993
(51) Int. Cl.: C09B 48/00, C07D 471/04, C07C 67/343, C07C 249/02

(54) **PIGMENT MANUFACTURING PROCESS**
PIGMENTHERSTELLUNGSVERFAHREN
PROCEDE DE FABRICATION DE PIGMENTS

(30) Priority: 04.11.1992 GB 9223048
(43) Date of publication of application: 23.08.1995
(73) Proprietor: ZENECA LIMITED, London W1Y 6LN (GB)
(72) Inventor: PIKE, Leslie Clark, Clackmannan, Clackmannanshire FK10 4JJ (GB); WOOLLVEN, Peter, Kirkliston, West Lothian EH29 9BJ (GB)
(74) Representative: Giles, David Eric
(86) International application number: GB9302157
(87) International publication number: WO9410249

(56) References cited:
- EP-A- 0 005 375
- EP-A- 0 057 873
- EP-A- 0 235 647
- EP-A- 0 536 083
- DE-A- 3 605 597
- US-A-44 355 589
- PATENT ABSTRACTS OF JAPAN vol. 11, no. 159 (C-423)(2606) 22 May 1987 & JP,A,61 286 343 (NIPPON SHOKUBAI KAGAKU KOGYO CO. LTD.) 16 December 1986 cited in the application
- PATENT ABSTRACTS OF JAPAN vol. 6, no. 24 (C-91)(902) 12 February 1982 & JP,A,56 147 749 (UBE KOSAN K. K.) 16 November 1981
- PATENT ABSTRACTS OF JAPAN vol. 11, no. 317 (C-452)(2764) 15 October 1987 & JP,A,62 106 062 (NIPPON SHOKUBAI KAGAKU KOGYO CO. LTD.) 16 May 1987

## Description

This invention relates to a pigment manufacturing process, and more particularly to a process for preparing quinacridone and substituted quinacridones. Quinacridone has the structural Formula (I):

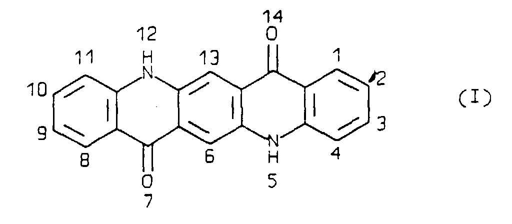

The numbers surrounding Formula (I) show the numbering system used to indicate the position of substituents in substituted quinacridones.

A variety of methods have been proposed for the manufacture of quinacridone. Scheme A below shows the chemical steps involved in one such method.

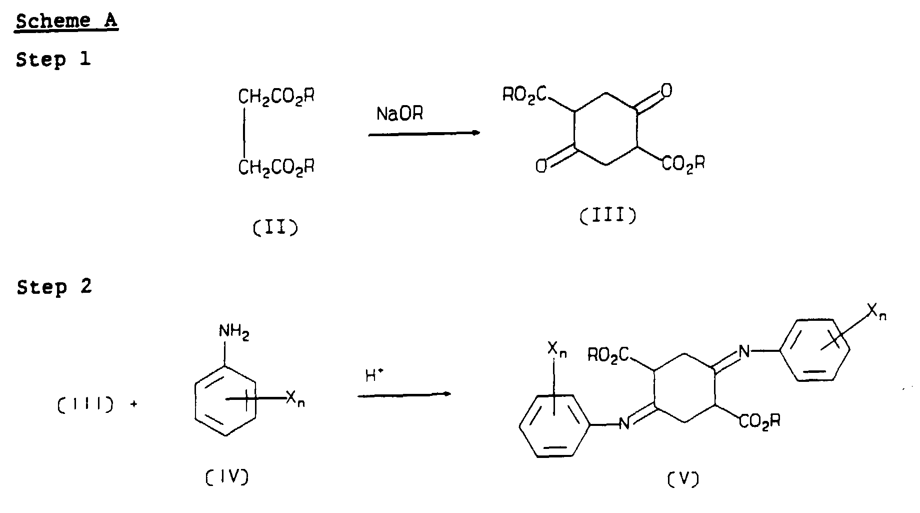

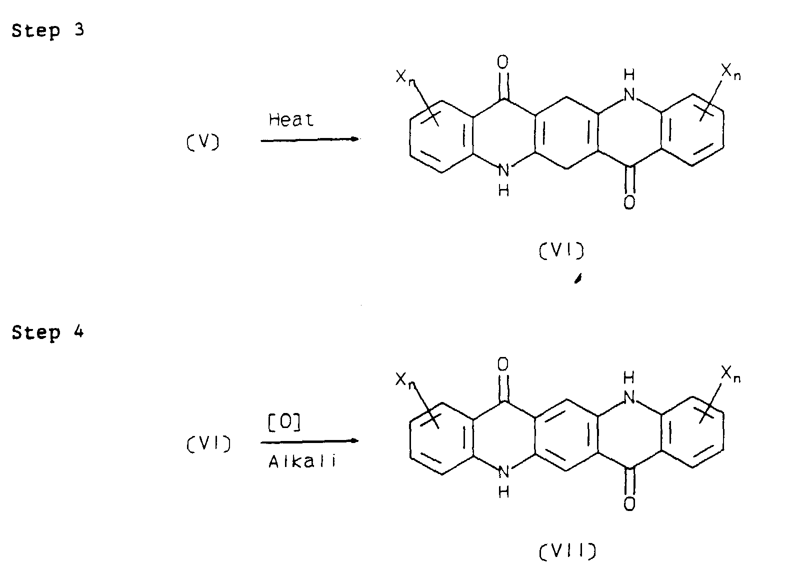

In Scheme A, the group R represents an alkyl group (e.g. c₁₋₃-alkyl, for example a methyl group), X is methyl, chloro or fluoro and n is 0, 1 or 2. When n is 2, the substituents X may be the same or different.

According to Scheme A, a dialkylsuccinate (II) is treated with a sodium alkoxide in a solvent or diluent to form the cyclic dialkyl succinylsuccinate (III). Usually the succinate ester (II) is dimethyl succinate, and the sodium alkoxide is sodium methoxide.

In this case, the compound (III) will be dimethyl succinylsuccinate, hereinafter referred to as DMSS. In Step 2 of Scheme A, the cyclic succinylsuccinate (III) is reacted with an optionally substituted aniline (IV) in an acidic medium to give the dihdyrodianilinoterephthalic ester (V). In Step 3 of Scheme A, the ester (V) is heated in a diluent or solvent to convert it into the dihydroquinacridone (VI). In Step 4 of Scheme A, the dihydroquinacridone (VI) is treated with an oxidising agent to convert it into the quinacridone (VII).

With regard to Step 1 of Scheme A, it has been proposed to perform the reaction in the following reaction media: ethers, hydrocarbons, alcohols or dimethylsulphoxide (Japanese Patent Application 69127,577); benzene (J. Indian Chem. Soc. 1964); aliphatic ethers or dioxan (UK Patent 751,035); excess of the dialkylsuccinate (German Patent Application 3423548); methanol (US Patent 4,435,589); or an aromatic hydrocarbon containing a proportion of polyethylene glycol, a monoether thereof, or diglyme (Japanese Patent Application 61-286343).

With regard to Step 2 of Scheme A, it has been proposed to perform the reaction in an alcohol (German Patent Application 3,104,644).

With regard to Step 3 of Scheme A, it has been proposed to use the following substances as the medium for the reaction: Dowtherm or Thermex, (Dowtherm and Thermex are Registered Trade Marks) which are mixtures of diphenyl oxide and biphenyl, a methylnaphthalene, diethylphthalate, or a mineral oil boiling within the range 225-300°C (US Patent 2,821,530); benzene in an autoclave (Japanese Patent Application 44/3216); a ditolyl ether (German Patent Application 3,605,976); syrupy phosphoric acid (e.g. US Patent 3,072,660); a methylnaphthalene, diethylphthalate, or a mineral oil (US Patent 3,009,916); or N-methylpyrrolidone (Japanese Patent Application 45/16340).

With regard to Step 4, it has been proposed to carry out the oxidation in a reaction medium comprising a mixture of aqueous sodium hydroxide with ethanol, acetone or ethylene glycol (US Patent 2,821,529); in an alcohol, glycerol, a 2-alkoxyethanol, ethanolamine or dioxan (US Patent 2,969,366); dimethyl sulphoxide (Japanese Patent Application 56145512); sulpholane (French Patent Application 1,496,960); or Dowtherm (Dowtherm is a Registered Trade Mark) and chloronaphthalene (German Patent Application 3,515,875).

It has furthermore been proposed to carry out Steps 1, 2 and 3 consecutively without isolating the products (III) and (IV) formed in Steps 1 and 2 before proceeding with Step 3 (US Patent 2,821,529). It has also been proposed to carry out Steps 2 and 3 consecutively without isolating the product (IV) from Step 2 before proceeding with Step 3 (US Patent 2,821,530). In UK Patent 894610 it has been proposed to perform the cyclisation reaction of Step 3 using polyphosphoric acid as the reaction medium, and then to dilute the reaction mixture with water and oxidise the dihydro quinacridone (VI) in situ to the quinacridone (I).

It has now been discovered that certain aliphatic polyethers are suitable for use as a reaction medium in each of the Steps 1 to 4 of Scheme A above. The aliphatic polyethers include those having the formula R¹[O(CH₂)ₘ]ₙOR¹ wherein R¹ is alkyl of 1 to 4 carbon atoms, m is 2 or 3, and n is 1, 2, 3 or 4. Particular examples of these polyethers include the following:

| | |
|---|---|
| CH₃OCH₂CH₂OCH₃ | glyme |
| CH₃(OCH₂CH₂)₂OCH₃ | diglyme |
| CH₃(OCH₂CH₂)₃OCH₃ | triglyme |
| CH₃(OCH₂CH₂)₄OCH₃ | tetraglyme |
| C₄H₉(OCH₂CH₂)₂OC₄H₉ | butyldiglyme |

As compared with materials previously used as reaction media for the processes outlined in Scheme A, the aliphatic polyethers have the advantage of lower toxicity. They are biodegradable and many are miscible with water.

Mixtures of different aliphatic polyethers may be used as the reaction medium if desired. Where the steps of Scheme A are carried out separately, with the product of each step being isolated before use in the next step, the aliphatic polyether used as the reaction medium need not be the same one in each step. However, since the aliphatic polyethers are in general suitable as reaction media for each step of Scheme A, it is possible if desired to carry out two or more of the steps successively in the same reaction medium without isolating the intermediate product produced in each step. Thus Steps 1 and 2, 2 and 3, or 3 and 4 of Scheme A can be combined, or Steps 1 to 3 or even all four steps may be performed without isolating the intermediates. When performing Step 4 it is preferred to add a co-solvent, for example a 2-alkoxyethanol or an ethanolamine, to the reaction medium.

According to the present invention, therefore, there is provided a process of preparing a quinacridone of Formula (VII): 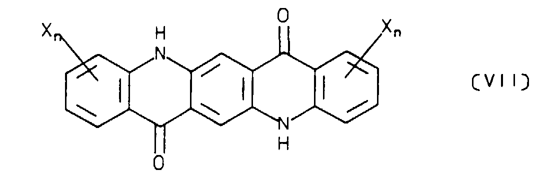 wherein X is methyl, chloro or fluoro, and n is 0, 1 or 2, which comprises:
(a) treating a dialkylsuccinate of Formula (II): 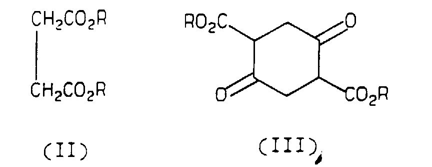 wherein R is alkyl of 1 to 3 carbon atoms, with at least the stoichiometric amount of a solution of a sodium alkoxide NaOR in an alcohol ROH, in a reaction medium principally comprising one or more aliphatic polyethers of the formula R¹[O(CH₂)ₘ]ₙOR¹ wherein R¹ is C₁₋₄-alkyl, m is 2 or 3, and n is 1, 2, 3 or 4, at a temperature of 70°C to 120°C, and maintaining the reaction mixture at a temperature of from 90°C to 150°C for a period sufficient to complete the formation of the dialkylsuccinylsuccinate (III), cooling the reaction mixture;
(b) adding an aniline of Formula (IV): 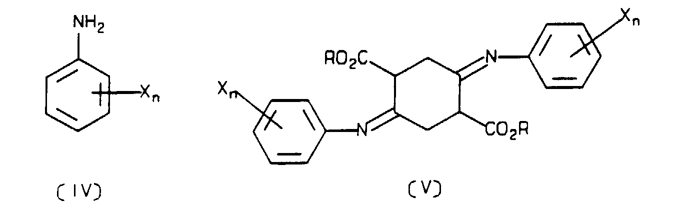 wherein X is methyl, chloro or fluoro, and n is 0, 1 or 2, acidifying the reaction mixture, heating it to 60°C to 100°C to convert the dialkylsuccinylsuccinate (III) in to the dihydro dianilinoterephthalic ester (V) wherein X and n are as defined for Formula (IV), cooling the reaction mixture and neutralising it;
(c) heating the reaction mixture under a vacuum to distil off water, alcohol ROH, and any unreacted aniline, placing the reaction mixture under an inert atmosphere, optionally adding more aliphatic polyether, and heating the reaction mixture to a temperature of 225-300°C, whereby the dihydro dianilinoterephthalic ester (V) is converted to the dihydroquinacridone (VI); and
(d) adding a mono-, di- or tri-ethanolamine or a 2-alkoxyethanol of formula R²OCH₂CH₂OH wherein R² is C₁₋₄-alkyl, making the reaction mixture alkaline, oxidising the dihydroquinacridone (VI) to form the quinacridone (VII), and recovering the quinacridone from the reaction mixture.

In a variation of the foregoing process, the dihydroquinacridone (VI) is separated from the reaction mixture after its formation in step (c), and is then oxidised to the quinacridone (VII) in a reaction medium comprising a 2-alkoxyethanol (e.g. 2-ethoxyethanol or 2-methoxyethanol) and aqueous sodium hydroxide.

In a further aspect, the invention provides a process of preparing a dihydrodianilinoterephthalic ester of Formula (V) wherein R is alkyl of 1 to 3 carbon atoms, X is methyl. chloro or fluoro and n is 0, 1 or 2, which comprises heating a dialkylsuccinylsuccinate of Formula (III, R=C₁₋₃-alkyl) with an aniline of Formula (IV), wherein X and n are defined as above for Eormula (V) in the presence of an acid, in a reaction medium comprising one or more aliphatic polyethers of the formula defined above, together with an alcohol of formula ROH, at a temperature of 60-100°C for a period sufficient to complete formation of the ester of Formula (V), and recovering the ester of Formula (V).

In a further aspect, the invention provides a process of preparing a dihydroquinacridone of Formula (VI), wherein X is methyl, chloro or fluoro and n is 0, 1 or 2, which comprises heating under non-oxidising conditions a dialkyldihydrodianilinoterephthalic ester of Formula (V), wherein X and n are defined as for Formula (VI) above, and R is C₁₋₃-alkyl, in a reaction medium comprising one or more aliphatic polyethers of the formula defined above, to a temperature from about 225-300°C, and separating the dihydroquinacridone so formed from the reaction mixture. Preferably the reaction mixture is heated to a temperature of 235-280°C. Preferably the process is carried out under an inert atmosphere, for example an atmosphere of nitrogen. The dihydroquinacridone produced in the reaction is insoluble in the reaction medium and may be recovered at the end of the reaction by filtering it off.

The invention further provides a process of preparing a dihydroquinacridone of Formula (VI), as described in the last foregoing paragraph, wherein the reaction medium additionally comprises a strong non-nucleophilic base. The inclusion of such a base can improve the yield of dihydroquinacridone (VI). The amount of base used is preferably equal in weight to the dialkyldihydrodianilinoterephthalic ester of Formula (V) but the exact amount is not critical, and more or less may be used if desired. The base used is preferably one which has a relatively high boiling point, that is to say a boiling-point which approaches or is equal to or greater than the temperature at which the reaction is carried out, since a base of low boiling point would be volatilised from the reaction mixture, with consequent loss of its effect. A suitable strong non-nucleophilic base is 1,8-bis (dimethyl amino)naphthalene, which is commonly referred to as "Proton Sponge".

The oxidation of the dihydroquinacridone (VI) may be brought about by a variety of oxidising agents, and the choice of oxidising agent is not critical. The oxidising agent may be for example, nitrobenzene, sodium m-nitrobenzenesulphonate, sodium polysulphide or oxygen. The alkaline reaction medium may be for example a mixture of one or more aliphatic polyethers as hereinbefore defined with an aqueous solution of an alkali, for example aqueous sodium hydroxide. The oxidation is preferably conducted at an elevated temperature, for example 100-160°C. The quinacridone (VII) produced in the process may be recovered from the reaction mixture by filtering it off, it may then be washed with an alcohol (e.g. methanol) to remove organic impurities, and then washed with water and dried if required.

Examples of quinacridones which may be prepared by the process of the invention include 2,9-dimethylquinacridone, 2,9-dichloroquinacridone, 3,10-dichloroquinacridone, difluoroquinacridones, and unsubstituted quinacridone in its beta or gamma form. The invention is illustrated by the following Examples.

### Example 1

This Example illustrates the preparation of dimethyl succinylsuccinate (also referred to as DMSS) having the Formula (III, R=Me) in tetraglyme, an aliphatic polyether reaction medium, according to the invention.

To a reactor is charged tetraglyme (400 kg) followed by dimethyl succinate (468 kg). A nitrogen blanket is applied and the mixture is heated to 100°C. Sodium methoxide liquor 27% w/w (716 kg) is added slowly while the methanol is allowed to distil out. This is condensed for use later. The mixture is held at 110°C for 5 hours and cooled to approximately 30°C. A Solution of sulphuric acid in water is added to liberate the free DMSS and the mixture is filtered. The product is washed with water until acid free and dried.

The yield is 266 kg of dimethyl succinylsuccinate, equivalent to 73% theory.

### Example 2

This Example illustrates the preparation of DMSS Formula (III, R-Me) in diglyme, an aliphatic polyether reaction medium, according to the invention.

Diglyme (400 kg) followed by dimethyl succinate (468 kg) is charged to a reactor under an atmosphere of nitrogen. The mixture is heated to 100°C and sodium methoxide liquor 27% w/w (716 kg) is added slowly while the methanol is allowed to distil out, and is condensed and collected for later use. The mixture is held at 110°C for 5 hours and then cooled to about 30°C. A solution of sulphuric acid in water is added to liberate the DMSS and the mixture is filtered. The product is washed with water until free of acid and dried.

The yield is 266 kg of dimethyl succinylsuccinate, equivalent to 73% of theory.

### Example 3

This Example illustrates the preparation of dimethyldihydrodi-toluidinoterephthalic ester (also referred to herein as dimethyl DATE(M)) having the Formula (V, R=Me, X=4-Me) in an aliphatic polyether reaction medium, according to the invention.

To a reactor is added dimethyl succinylsuccinate (100 kg), methanol (270 1) and tetraglyme (200 1) followed by p-toluidine (154 kg) and sulphuric acid (20 kg). The mixture is heated to 76°C and stirred at this temperature for 5 hours. Water (300 1) is then added and the mixture is filtered. The product paste is washed with water and then stirred with dilute hydrochloric acid to remove unreacted toluidine, and then refiltered and washed acid-free with water and dried.

The yield is 170 kg of dimethyldihydroditoluidino terephthalic ester, equivalent to a yield of 96% theory.

### Example 4

This Example illustrates the preparation of dimethyl succinylsuccinate (DMSS, Formula III, R=CH₃) in an aliphatic polyether reaction medium, and its conversion into dimethyl dihydrotoluidino terephthalic ester (dimethyl DATE(M) having the Formula (V, R=Me, X=4-Me) in the same solvent.

Tetraglyme (400 kg) followed by dimethyl succinate (468 kg) is charged to a reactor under an atmosphere of nitrogen. The mixture is heated to 100°C. Sodium methoxide liquor 27% w/w (716 kg) is added slowly while the methanol is allowed to distil out. This is condensed for use later. The mixture is held at 110°C for 5 hours and cooled to approximately 30°C. The methanol is returned followed by p-toluidine (400 kg) followed by addition of concentrated sulphuric acid (270 kg), allowing the temperature to rise to reflux (73°C). The mixture is held at reflux for 5 hours. Cooling is applied and the batch is neutralised to pH 8 with sodium carbonate. The mixture is filtered and the paste is washed with water, methanol and finally with water again, and dried.

The yield is 455 kg of dimethyl DATE(M), equivalent to 70% theory.

### Example 5

This Example illustrates the preparation of 2,9-dimethyl-6,13-dihydroquinacridone (also referred to herein as DMDHQ), having the Formula (VI, X=Me in the 2 and 9 positions) in an aliphatic polyether reaction medium, according to the invention.

Tetraglyme (4000 kg), followed by dimethyldihydroditoluidinoterephthalic ester (400 kg) Formula (V, R=Me, X=4-Me) is charged to a reactor under an atmosphere of nitrogen. The vessel is heated to 277°C as fast as possible, and held there for 2 hours. During this time, some volatile material (methanol generated in the reaction plus some tetraglyme) distils out. The vessel is then cooled to 100°C. The mixture is filtered and the DMDHQ paste is dried.

The yield is 316 kg, equivalent to 94% of theory.

### Example 6

This Example illustrates the preparation of 2,9-dimethyl-6,13-dihydroquinacridone (DMDHQ) in a reaction medium comprising a mixture of aliphatic polyethers, according to the invention.

A mixture of triglyme (2 parts) and tetraglyme (1 part) (4000 kg), having a boiling point of 238°C is charged to a reactor under an atmosphere of nitrogen, followed by dimethyldihydroditoluidinoterephthalic ester Formula (V, R=Me, X=4-Me) (400kg). The vessel is heated to the initial boiling point of the solvent as quickly as possible, and held there for 2 hours. The vessel is then cooled to 100°C and methanol is added. The mixture is filtered and the DMDHQ paste is dried.

The yield is 146 kg, equivalent to 44% of theory.

### Example 7

This Example further illustrates the preparation of 2,9-dimethyl-6,13-dihydroquinacridone (DMDHQ). The procedure of Example 6 is followed, except that instead of the triglyme/tetraglyme mixture, butyldiglyme (having a boiling point of 257°C) is used as the reaction medium.

The yield of DMDHQ is 178 kg, equivalent to 53% of theory.

### Example 8

This Example further illustrates the preparation of DMDHQ. The procedure of Example 6 is followed, except that 1,8-bis(dimethyl amino)naphthalene (400kg) is charged to the reactor along with the reaction medium.

The yield of DMDHQ is 208 kg, equivalent to 62% of theory.

### Example 9

This Example illustrates the conversion of dimethyl succinylsuccinate (DMSS) Formula (III, R=Me) to dimethyldihydrodi-toluidinoterephthalic ester (DATE(M)) Formula (V, R=Me, X=4-Me) and the conversion of the latter compound in the same reaction medium to 2,9-dimethyl-6,13-dihydroquinacridone (DMDHQ).

Tetraglyme (2000 kg), DMSS (100 kg) and methanol (270 l) are charged to a reactor under an atmosphere of nitrogen, followed by p-toluidine (154 kg) and sulphuric acid (20 kg). The mixture is heated to 76°C and held there for 5 hours. Sodium carbonate solution in water is added to neutralise the acid and the reactor is then set up for vacuum distillation. A vacuum of 20 millibars is applied and the volatile components are distilled out, with methanol coming off at 25-30°C, water and p-toluidine with some tetraglyme between 30 and 140°C, and only tetraglyme above 150°C at this pressure. The temperature is not allowed to rise about 150°C until all the unreacted toluidine has been removed; failure to do this reduces the yield and quality of the product.

Nitrogen is admitted to the reactor to release the vacuum, and the reactor is heated to 277°C and kept there for 2 hours under a nitrogen atmosphere.

The reactor is then cooled to 50°C and the DMDHQ is filtered off, washed with methanol and with water, and dried.

The yield of DMDHQ is 120 kg, equivalent to 80% of theory.

### Example 10

This Example illustrates the preparation of 2,9-dimethylquinacridone (DMQ) by oxidation of 2,9-dimethyl-6,13-dihydroquinacridone (DMDHQ) in a reaction medium comprising 2-e.thoxyethanol. A mixture of 2-ethoxyethanol (6000 kg) and aqueous sodium hydroxide (47% w/w; 400 kg) is heated to 110°C under an atmosphere of nitrogen. DMDHQ (600 kg) and nitrobenzene (400 kg) are added, and the mixture is heated to 135°C and held there for 6 hours. The mixture is then cooled to below 100°C, diluted with about an equal volume of water, and filtered on a slurry filter. The DHQ is washed with methanol and then with water and dried. The yield of 2,9-dimethylquinacridone is 567 kg, equivalent to a yield of 95% of theory.

### Example 11

This Example illustrates the preparation of 2,9-dimethylquinacridone (also referred to herein as DMQ) by oxidising DMDHQ in a mixture of tetraglyme and 2-ethoxyethanol as the reaction medium.

2-Ethoxyethanol (1000 kg) is charged to a reactor maintained under an atmosphere of nitrogen, followed by sodium hydroxide solution in water (47% w/w; 400 kg), and the mixture is heated to 110°C to ensure complete dissolution of the sodium hydroxide. Tetraglyme (5000 kg) is then added, followed by DMDHQ (600 kg). Nitrobenzene (400 kg) is then added and the vessel is heated to 160°C and held there for 6 hours. The mixture is then cooled to below 100°C, diluted with water and filtered on a slurry filter. The 2,9-dimethylquinacridone paste is washed with methanol and then with water, and dried.

The yield of DMQ is 567 kg equivalent to 95% of theory.

### Example 12

This Example illustrates the preparation of DMQ by oxidising DMDHQ in a mixture of tetraglyme and triethanolamine as the reaction medium. The procedure of Example 11 is followed, except that triethanolamine (1000 kg) is used instead of 2-ethoxyethanol.

The yield of DHQ is 567 kg.

### Example 13

This Example illustrates the preparation of DMQ by oxidising DMDHQ with air in a mixture of tetraglyme and 2-ethoxyethanol as the reaction medium.

The procedure of Example 10 is followed, but instead of adding nitrobenzene, air is bubbled through the reaction mixture to oxidise the DMDHQ to DMQ.

The yield of DMQ is 567 kg.

### Example 14

This Example illustrates the preparation of 2,9-dimethylquinacridone from dimethyl succinate without isolation of the intermediate products.

Tetraglyme (400 kg) and dimethyl succinate (468 kg) are charged to a reactor vessel under an atmosphere of nitrogen. The mixture is heated to 100°C and sodium methoxide solution in methanol (27% w/w; 898 kg) is added slowly while the methanol is allowed to distil out, and is condensed for later use. The mixture is held at 110°C for 5 hours and cooled to about 30°C. The methanol is returned, followed by p-toluidine (400 kg), followed by concentrated sulphuric acid (270 kg) added slowly, the temperature being allowed to rise to reflux (73°C). The mixture is held at reflux for 5 hours. The mixture is cooled and brought to pH 8 with sodium carbonate. The mixture, containing dimethyldihydroditoluidinoterephthalic ester Formula (V, R=Me, X=4-Me) is now transferred, still under nitrogen, to a second vessel equipped for vacuum distillation and the temperature is raised to 140°C under a vacuum of 20 mm mercury, whereby methanol, excess of toluidine and water are distilled out, together with some tetraglyme. The vacuum is released with nitrogen, and more tetraglyme (3000 kg) is added. The vessel is heated to 277°C as quickly as possible, and held there for 2 hours. During this time some methanol and tetraglyme distils out and is collected for future use.

The vessel is then cooled to 100°C and the reaction mixture, now containing 2,9-dimethyl-6,13-dihydroquinacridone is transferred to a third vessel, previously purged with nitrogen, for the oxidation stage. In this vessel is previously prepared a mixture of 2-ethoxyethanol (400 kg) and aqueous sodium hydroxide (47% w/w, 400 kg), which is heated to 110°C to ensure that the sodium hydroxide is in solution. Following the transfer of the reaction mixture, nitrobenzene (246 kg) is added, and the vessel is heated to 160°C for 6 hours. The mixture is then cooled to below 100°C, diluted with water and filtered on a slurry filter. The filtrate may be distilled to recover the tetraglyme and 2-ethoxyethanol, and the distillation residue may be incinerated. The product paste containing the 2,9-dimethylquinacridone is washed with methanol and then with water, and dried if required. The methanol washes may be worked up to recover tetraglyme.

## Claims

1. A process of preparing a quinacridone of Formula (VII): 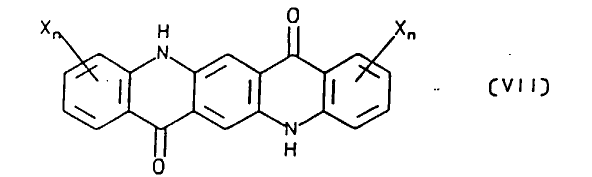 wherein X is methyl, chloro or fluoro, and n is 0, 1 or 2, which comprises:
(a) treating a dialkylsuccinate of Formula (II): 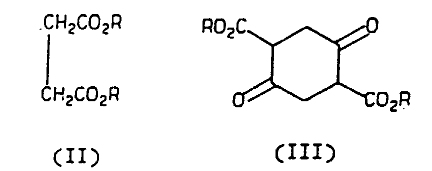 wherein R is alkyl of 1 to 3 carbon atoms, with at least the stoichiometric amount of a solution of a sodium alkoxide NaOR in an alcohol ROH, in a reaction medium principally comprising one or more aliphatic polyethers of the formula R¹[O(CH₂)ₘ]ₙOR¹ wherein R¹ is C₁₋₄-alkyl, m is 2 or 3, and n is 1, 2, 3 or 4, at a temperature of 70°C to 120°C, and maintaining the reaction mixture at a temperature of from 90°C to 150°C for a period sufficient to complete the formation of the dialkylsuccinylsuccinate (III), cooling the reaction mixture;
(b) adding an aniline of Formula (IV): 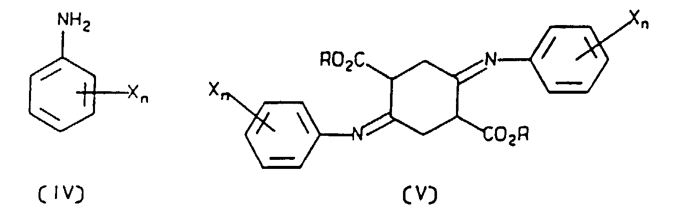 wherein X is methyl, chloro or fluoro, and n is 0, 1 or 2, acidifying the reaction mixture, heating it to 60°C to 100°C to convert the dialkylsuccinylsuccinate (III) in to the dihydro dianilinoterephthalic ester (V) wherein X and n are as defined for Formula (IV), cooling the reaction mixture and neutralising it;
(c) heating the reaction mixture under a vacuum to distil off water, alcohol ROH, and any unreacted aniline, placing the reaction mixture under an inert atmosphere, optionally adding more aliphatic polyether, and heating the reaction mixture to a temperature of 225-300°C, whereby the dihydro dianilinoterephthalic ester (V) is converted to the dihydroquinacridone (VI); and
(d) adding a mono-, di- or tri-ethanolamine or a 2-alkoxyethanol of formula R²OCH₂CH₂OH wherein R² is C₁₋₄-alkyl, making the reaction mixture alkaline, oxidising the dihydroquinacridone (VI) to form the quinacridone (VII), and recovering the quinacridone from the reaction mixture.

2. A process of preparing a dihydrodianilinoterephthalic ester of Formula (V) : 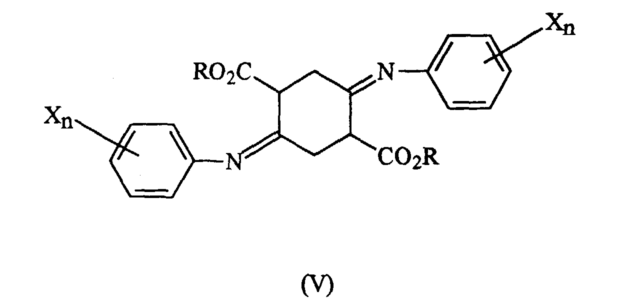 wherein R is alkyl of 1 to 3 carbon atoms, X is methyl, chloro, or fluoro, and n is 0, 1, or 2, which comprises heating a dialkylsuccinylsuccinate of Formula (III, R=C₁₋₃-alkyl) 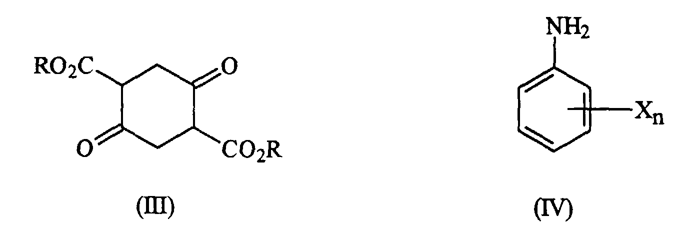 with an aniline of Formula (IV), wherein X is methyl, chloro, or fluoro, and n is 0, 1, or 2, in the presence of an acid in a reaction medium comprising one or more aliphatic polyethers of the formula R¹[O(CH₂)ₘ]ₙOR¹ wherein R¹ is C₁₋₄-alkyl, m is 2 or 3, and n is 1, 2, 3, or 4, together with an alcohol of formula ROH, at a temperature of 60-100°C for a period sufficient to complete formation of the ester of Formula (V) and recovering the ester of Formula (V).

3. A process of preparing a dihydroquinacridone of Formula (VI): 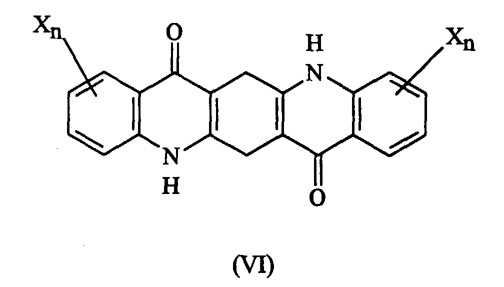 wherein X is methyl, chloro, or fluoro, and n is 0, 1, or 2, which comprises heating under non-oxidising conditions a dialkyldihydrodianilinoterephthalic ester of formula (V): 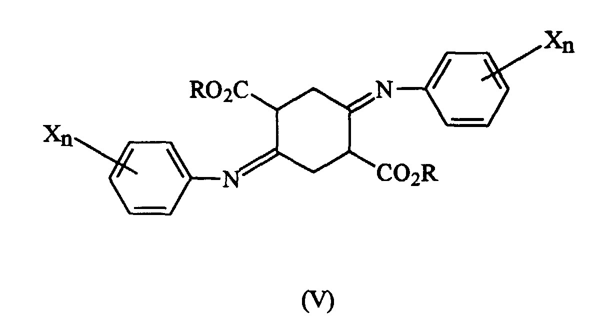 wherein X is methyl, chloro, or fluoro, n is 0, 1, or 2, and R is C₁₋₃-alkyl, in a reaction medium consisting principally of one or more aliphatic polyethers of the formula R¹[O(CH₂)ₘ]ₙOR¹ wherein R¹ is C₁₋₄-alkyl, m is 2 or 3, and n is 1, 2, 3, or 4, to a temperature from about 225-300°C, and separating the dihydroquinacridone so formed from the reaction mixture.

4. A process as claimed in claim 3, wherein the reaction medium further comprises a strong non-nucleophilic base.

## Patentansprüche

1. Verfahren zur Herstellung eines Chinacridons mit der Formel (VII): 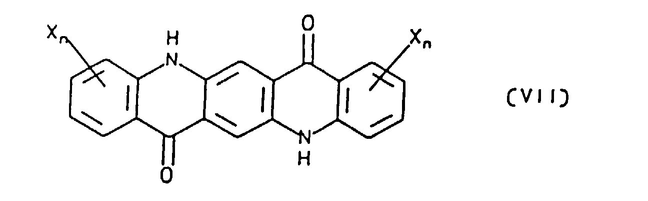 in der X für Methyl, Chlor oder Fluor steht und n 0, 1 oder 2 ist, bei dem:
(a) ein Dialkylsuccinat mit der Formel (II): 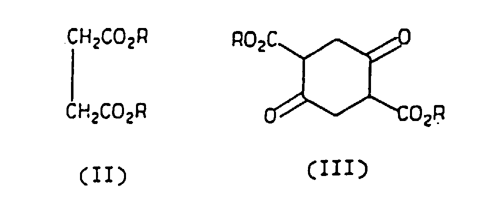 in der R für Alkyl mit 1 bis 3 Kohlenstoffatomen steht, mit mindestens der stöchiometrischen Menge einer Lösung eines Natriumalkoxids NaOR in einem Alkohol ROH in einem Reaktionsmedium behandelt wird, das hauptsächlich einen oder mehrere aliphatische Polyether mit der Formel R¹[O(CH₂)ₘ]ₙOR¹, in der R¹ für C₁₋₄-Alkyl steht, m 2 oder 3 ist und n 1, 2, 3 oder 4 ist, enthält, und zwar bei einer Temperatur von 70°C bis 120°C, wobei das Reaktionsgemisch ausreichend lange bei einer Temperatur von 90°C bis 150°C gehalten wird, um die Bildung des Dialkylsuccinylsuccinats (III) zu vervollständigen, worauf das Reaktionsgemisch abgekühlt wird,
(b) ein Anilin mit der Formel (IV) : 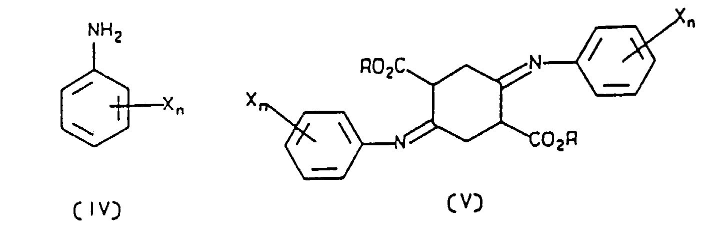 in der X für Methyl, Chlor oder Fluor steht und n 0, 1 oder 2 ist, dazugegeben wird, das Reaktionsgemisch angesäuert wird, auf 60°C bis 100°C angesäuert wird, um das Dialkylsuccinylsuccinat (III) in den Dihydrodianilinoterephthalsäureester (V), wobei X und n wie in Formel (IV) definiert sind, umzuwandeln, und das Reaktionsgemisch abgekühlt und neutralisiert wird,
(c) das Reaktionsgemisch im Vakuum zur Abdestillation von Wasser, Alkohol ROH und nicht umgesetztem Anilin erhitzt wird, das Reaktionsgemisch unter eine Inertatmosphäre gegeben wird, gegebenenfalls weiterer aliphatischer Polyether dazugegeben und das Reaktionsgemisch auf eine Temperatur von 225 - 300°C erhitzt wird, wodurch der Dihydrodianilinoterephthalsäureester (V) in das Dihydrochinacridon (VI) umgewandelt wird, und
(d) ein Mono-, Di- oder Triethanolamin oder ein 2-Alkoxyethanol mit der Formel R²OCH₂CH₂OH, in der R² für C₁₋₄-Alkyl steht, dazugegeben wird, das Reaktionsgemisch alkalisch gemacht wird, das Dihydrochinacridon (VI) zur Bildung des Chinacridons (VII) oxidiert wird und das Chinacridon aus dem Reaktionsgemisch gewonnen wird.

2. Verfahren zur Herstellung eines Dihydrodianilinoterephthalsäureesters mit der Formel (V): 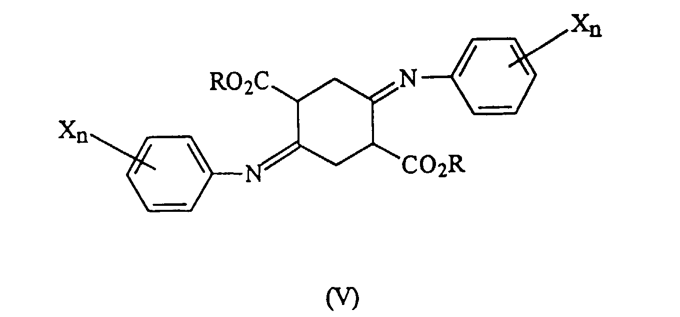 in der R für Alkyl mit 1 bis 3 Kohlenstoffatomen steht, X für Methyl, Chlor oder Fluor steht und n 0, 1 oder 2 ist, bei dem ein Dialkylsuccinylsuccinat mit der Formel (III, R=C₁₋₃-Alkyl): 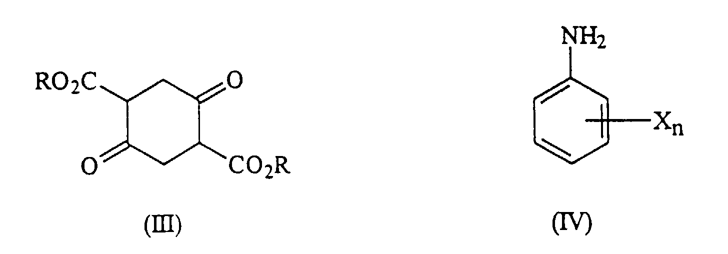 mit einem Anilin mit der Formel (IV), wobei X für Methyl, Chlor oder Fluor steht und n 0, 1 oder 2 ist, in Gegenwart einer Säure in einem Reaktionsmedium erhitzt wird, das einen oder mehrere aliphatische Polyether mit der Formel R¹[O(CH₂)ₘ]ₙOR¹, in der R¹ für C₁₋₄-Alkyl steht, m 2 oder 3 ist und n 1, 2, 3 oder 4 ist, enthält, und zwar zusammen mit einem Alkohol mit der Formel ROH und bei einer Temperatur von 60 - 100°C und ausreichend lange, um die Bildung des Esters mit der Formel (V) zu vervollständigen, worauf der Ester mit der Formel (V) gewonnen wird.

3. Verfahren zur Herstellung eines Dihydrochinacridons mit der Formel (VI): 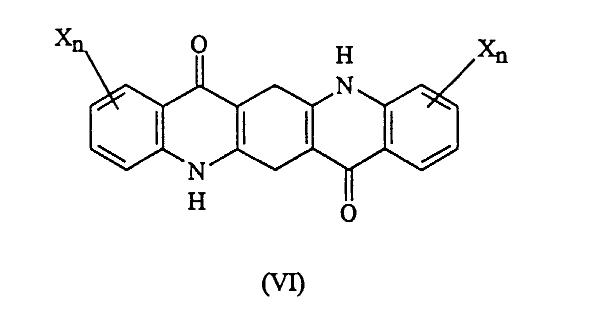 in der X für Methyl, Chlor oder Fluor steht und n 0, 1 oder 2 ist, bei dem unter nicht oxidierenden Bedingungen ein Dialkyldihydrodianilinoterephthalsäureester mit der Formel (V): 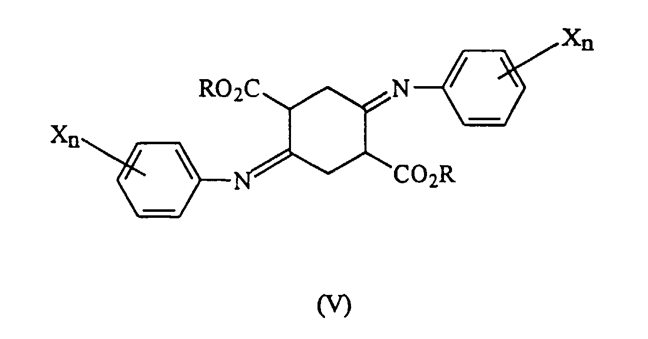 in der X für Methyl, Chlor oder Fluor steht und n 0, 1 oder 2 ist und R für C₁₋₃-Alkyl steht, in einem Reaktionsmedium erhitzt wird, das hauptsächlich ein oder mehrere aliphatische Polyether mit der Formel R¹[O[CH₂)ₘ]ₙOR¹, in der R¹ für C₁₋₄-Alkyl steht, m 2 oder ist und n 1, 2, 3 oder 4 ist, enthält, und zwar auf eine Temperatur von etwa 225 - 300°C, worauf das so gebildete Dihydrochinacridon aus dem Reaktionsgemisch abgetrennt wird.

4. Verfahren nach Anspruch 3, wobei das Reaktionsmedium außerdem eine starke nicht-nukleophile Base enthält.

## Revendications

1. Procédé de préparation d'une quinacridone de formule (VII) ; 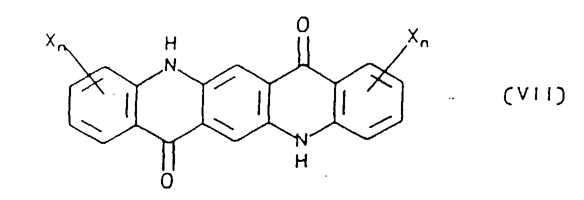 dans laquelle X représente un groupe méthyle, chloro ou fluoro et n est égal à 0, 1 ou 2, qui comprend :
(a) le traitement d'un succinate de dialkyle de formule (II) : 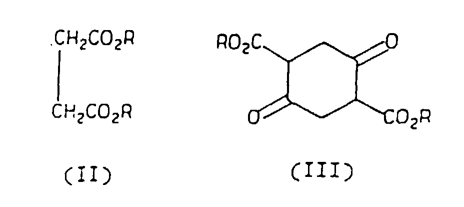 dans laquelle R représente un groupe alkyle ayant 1 à 3 atomes de carbone, avec au moins la quantité stoechiométrique d'une solution d'un alcoolate de sodium de formule NaOR dans un alcool de formule ROH, dans un milieu réactionnel comprenant principalement un ou plusieurs polyéthers aliphatiques de formule R¹[O(CH₂)ₘ]ₙOR¹ dans laquelle R¹ représente un groupe alkyle en C₁ à C₄, m est égal à 2 ou 3, et n est égal à 1, 2, 3 ou 4, à une température de 70°C à 120°C, et le maintien du mélange réactionnel à une température de 90°C à 150°C pendant un temps suffisant pour achever la formation du dialkylsuccinylsuccinate (III), puis le refroidissement du mélange réactionnel ;
(b) l'addition d'une aniline de formule (IV) : 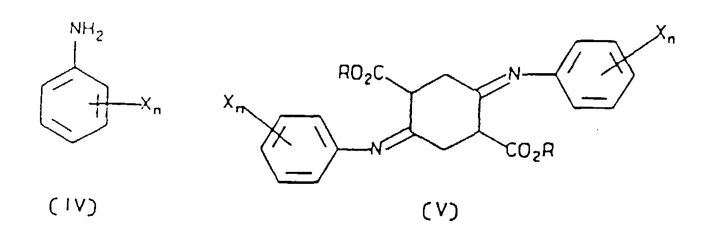 dans laquelle X représente un groupe méthyle, chloro ou fluoro et n est égal à 0, 1 ou 2, l'acidification du mélange réactionnel, le chauffage de ce mélange réactionnel à une température de 60°C à 100°C pour la transformation du dialkylsuccinylsuccinate (III) en l'ester dihydrodianilinotéréphtalique (V) dans lequel X et n répondent aux définitions mentionnées pour la formule (IV), le refroidissement du mélange réactionnel et la neutralisation de ce mélange réactionnel ;
(c) le chauffage du mélange réactionnel sous vide pour éliminer par distillation l'eau, l'alcool de formule ROH et toute quantité d'aniline n'ayant pas réagi, la mise en place du mélange réactionnel sous une atmosphère inerte, facultativement l'addition d'une quantité supplémentaire de polyéther aliphatique, et le chauffage du mélange réactionnel à une température de 225 à 300°C, ce qui provoque la transformation de l'ester dihydrodianilinotéréphtalique (V) en la dihydroquinacridone (VI) ; et
(d) l'addition d'une mono-, di- ou triéthanolamine ou d'un 2-alkoxyéthanol de formule R²OCH₂CH₂OH dans laquelle R² représente un groupe alkyle en C₁ à C₄, la transformation du mélange réactionnel en un mélange réactionnel alcalin, l'oxydation de la dihydroquinacridone (VI) pour former la quinacridone (VII), et la séparation de la quinacridone du mélange réactionnel.

2. Procédé de préparation d'un ester dihydrodianilinotéréphtalique de formule (V) : 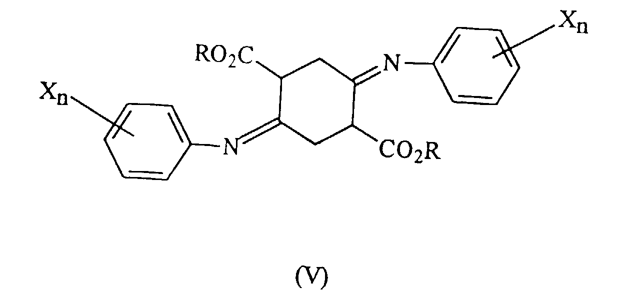 dans laquelle R représente un groupe alkyle ayant 1 à 3 atomes de carbone, X représente un groupe méthyle, chloro ou fluoro et n est égal à 0, 1 ou 2, qui comprend le chauffage d'un dialkylsuccinylsuccinate de formule (III, R=alkyle en C₁ à C₃) 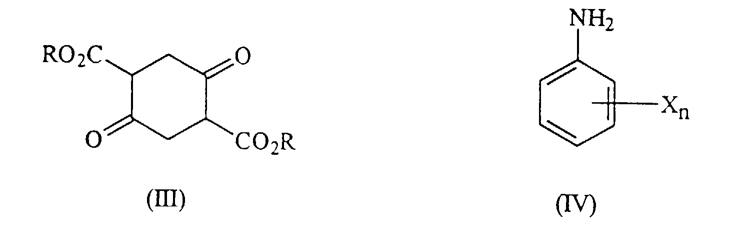 avec une aniline de formule (IV), dans laquelle X représente un groupe méthyle, chloro ou fluoro, et n est égal à 0, 1 ou 2, en présence d'un acide dans un milieu réactionnel comprenant un ou plusieurs polyéthers aliphatiques de formule R¹[O(CH₂)ₘ]ₙOR¹ dans laquelle R¹ représente un groupe alkyle en C₁ à C₄, m est égal à 2 ou 3, et n est égal à 1, 2, 3 ou 4, conjointement avec un alcool de formule ROH, à une température de 60 à 100°C pendant un temps suffisant pour achever la formation de l'ester de formule (V) et la séparation de l'ester de formule (V).

3. Procédé de préparation d'une dihydroquinacridone de formule (VI) : 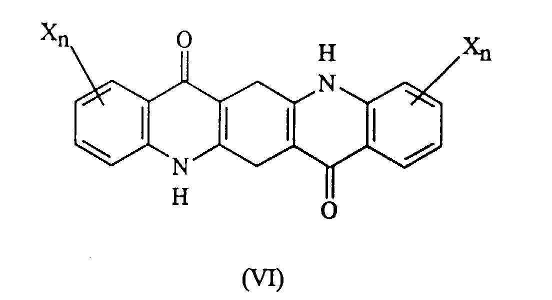 dans laquelle X représente un groupe méthyle, chloro ou fluoro et n est égal à 0, 1 ou 2, qui comprend le chauffage, dans des conditions non oxydantes, d'un ester dialkyldihydrodianilinotéréphtalique de formule (V) : 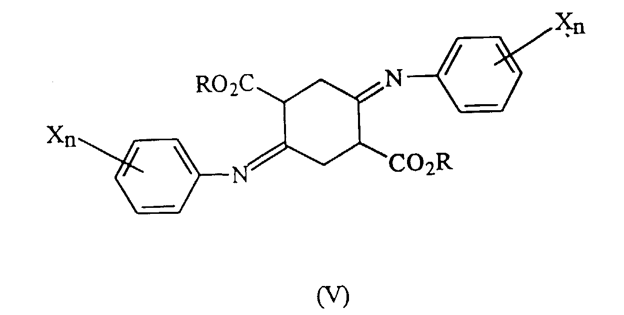 dans laquelle X représente un groupe méthyle, chloro ou fluoro, n est égal à 0, 1 ou 2 et R représente un groupe alkyle en C₁ à C₃, dans un milieu réactionnel consistant principalement en un ou plusieurs polyéthers aliphatiques de formule R¹[O(CH₂)ₘ]ₙOR¹ dans laquelle R¹ représente un groupe alkyle en C₁ à C₄, m est égal à 2 ou 3, et n est égal à 1, 2, 3 ou 4, à une température d'environ 225 à 300°C, et la séparation de la dihydroquinacridone, ainsi formée, du mélange réactionnel.

4. Procédé suivant la revendication 3, dans lequel le milieu réactionnel comprend en outre une base non nucléophile forte.
